# EUROPEAN PATENT SPECIFICATION

(11) **EP 2 734 167 B1**
(45) Date of publication and mention of the grant of the patent: **04.04.2018**
(21) Application number: 12815108.1
(22) Date of filing: 29.06.2012
(51) Int. Cl.: A61F 13/532, A61F 13/64, A61F 13/537, A61F 13/56, A61F 13/53

(54) **ABSORBENT PAD WITH ASYMMETRICAL ABSORPTION CAPACITY AND ABSORBENT ARTICLE COMPRISING THE SAME**
SAUGFÄHIGES KISSEN MIT ASYMMETRISCHER ABSORPTIONSKAPAZITÄT UND SAUGFÄHIGER ARTIKEL DAMIT
SERVIETTE ABSORBANTE AVEC CAPACITÉ D'ABSORPTION ASYMÉTRIQUE ET ARTICLE ABSORBANT COMPRENANT LADITE SERVIETTE

(30) Priority: 18.07.2011 CN 201110215113
(43) Date of publication of application: 28.05.2014
(73) Proprietor: Sca Hygiene Products AB, 405 03 Göteborg (SE)
(72) Inventor: WANG, Mandy Qin, Shanghai 201114 (CN); LOH, Johwin, Shanghai 201114 (CN); CARLBARK, Olle, Shanghai 201114 (CN); RÖNNBERG, Peter, S-405 03 Göteborg (SE)
(74) Representative: Valea AB
(86) International application number: PCT/SE2012/050740
(87) International publication number: WO 2013/012375

(56) References cited:
- JP-A- 2003 180 721
- US-A- 4 381 782
- US-A- 4 673 403
- US-A- 5 135 522
- US-A- 5 156 902
- US-A- 5 156 902
- US-A1- 2005 143 709

## Description

### TECHNICAL FIELD

The present invention relates to an absorbent pad and an absorbent article comprising the absorbent pad, in particular, to an absorbent article including a symmetrical absorbent pad with asymmetrical absorption capacity and a separate belt releasably attached to the absorbent pad.

### TECHNICAL BACKGROUND

It is known that absorbent articles, in the forms of diapers, pant diapers, napkins, or incontinent-protecting products, are generally used for babies and incontinent adults for the absorption of bodily exudates, such as blood, urine, sweat and faeces. In some cases, the absorbent articles are provided with belts to be placed around the waist of the wearer in order to facilitate putting on and putting off of the articles. In particular, the belt is designed in the form of a separate belt releasably attached to an absorbent pad, which is disclosed, for example, in WO 99/21522, WO94/26224, WO94/26222, WO94/26225 and US 4964860.

In various absorbent pads attached to the above belts, pads with different absorption capacity are used for different applications or different persons. Even the same person may appreciate that a pad has different absorption capacity in different portions of the pad. Various absorbent articles exhibiting regions with different absorption capacity have been developed.

US5156902 discloses an absorbent article in which a greater proportion of SAM (superabsorbent polymeric material) may be concentrated towards at least one longitudinal end of the absorbent body. It is also defined that 50-90 wt% of the SAM is located in the front half of the absorbent body. The article is limited to be used in one orientation.

US4673402 discloses a layered absorbent article wherein the core comprises an upper fluid acquisition/distribution layer with maximum 8 wt% of hydrogel material and a lower fluid storage layer comprising 9-60 wt% of hydrogel material. 75 wt% of the hydrogel material in the lower layer is located in the front two-thirds section of the absorbent article. The core is thus asymmetrical in thickness which means that the orientation for use is limited.

US4381782 discloses an absorbent fibrous structure, such as diaper. The absorbent core is, before being folded, substantially symmetrical in both the longitudinal direction and the transverse direction. The core is cut and folded to a desired asymmetrical shape thereby providing a thicker front portion exhibiting a higher absorption capacity and a thinner rear portion exhibiting a lower absorption capacity.

It can be seen from the above documents that the disclosed articles may have different absorption capacity along the longitudinal direction, i.e. higher absorption capacity towards any of the ends of the article. However, these articles can, when used in the intended manner, be placed on the wearer in one orientation only, either forward or rearward.

SE9702159 discloses a symmetrical article with different absorption capacity in its two halves, but with an asymmetrical absorption core, wider in one of its ends. In the description it is said that, due to the tapered profile, the two ends of the article has different absorption capacity. It is also mentioned that for menstruating women, there is a need for more absorption capacity at the rear end of the article during night and at the front end during day and that the article can be used at both these occasions. However, such an asymmetrical absorption core makes the article less comfortable.

US5135522 discloses a belt article comprising a multi-layered absorbent structure. It is disclosed that male infants tend to urinate further towards the front and female infants more in the centre of the crotch portion. It is further disclosed that the placement of the lower layer can be selected to best correspond with the actual target zone.

US 5135522 discloses an article with different absorption capacity in the two halves, but which can be oriented on the wearer by either of its ends facing forward or rearward. However, the higher absorption capacity in one of the halves is obtained by the presence of a second absorption core 74 in that half, that is to say, the different absorption capacity is obtained by different thickness in two longitudinal halves of the core. It is mentioned in the description of US 5135522 that "the use and placement of the discrete pledget 74 meet the absorbent capacity needs of a given user; the placement of the hydrogel-containing discrete pledget 74 more efficiently utilizes the absorbent capacity of the core 18 in the front one-half of the diaper 10".

One problem with such an absorption core asymmetrical in thickness is that the thicker half makes the article less comfortable, especially when it is oriented with one of its ends in one direction, e.g. with its thicker half in rearward direction.

Another problem is that the article is too noticeable when oriented with one of its ends in one direction (with its thicker half in reward direction).

### SUMMARY OF THE INVENTION

In view of the above disadvantages, an object of the invention is to provide an absorbent article which is geometrically symmetrical so that there is no limitation to the orientation of the absorbent article when placed on the wearer for the intended purpose, but with different absorption capacity in different portions in order to satisfy specific use requirements. That is to say, the article should, from a geometrical point of view, be symmetrical in longitudinal direction and in transverse direction and also have the same thickness on the same distance from the transversal centre line (measured perpendicular to the transversal centre line) before the core is wetted, but with higher absorption capacity in one of its longitudinal halves, so that the article can be attached to the user via a separate belt with any of its two halves in either forward or reward direction depending on the specific needs of the user.

To achieve said object, the present invention provides an absorbent pad having a chassis comprising a liquid-permeable topsheet and a liquid-impermeable backsheet, wherein the absorbent pad is geometrically symmetrical along its longitudinal and transversal centre lines and wherein the absorbent pad is provided with attaching arrangements (fastening means) by which the pad can be releasably attached to a separate belt adapted to be placed around the waist of the user, and wherein the absorbent pad further comprises an absorbent core interposed between the topsheet and the backsheet, which absorbent core is also geometrically symmetrical along the longitudinal and transversal centre lines of the pad and the absorbent core is divided into a first longitudinal half and a second longitudinal half along its longitudinal length, and wherein the absorbent core has a thickness which is substantially the same on the same distance from the transversal centre line measured perpendicular to the transversal centre line, before the core is wetted (i.e. in dry state), and wherein the first longitudinal half has a higher absorption capacity than the second longitudinal half.

In specific embodiments of the present invention, the absorption capacity ratio of the first longitudinal half to the second longitudinal half of the absorbent core is 80:20, in particular 60:40 and more particularly 55:45. In further specific embodiments of the present invention, the absorption capacity ratio of the first longitudinal half to the second longitudinal half of the absorbent core is 75:25, 70:30 or 65:35.

In the absorbent pad according to the present invention, the absorbent core comprises superabsorbing polymeric material, also called SAP. In a specific embodiment, the first longitudinal half contains a different amount of SAP than the second longitudinal half, which causes the two longitudinal halves to exhibit different absorption capacity. As further explained below, the difference in the dimensions of the absorbent core measured on equal perpendicular distances from the transversal centre line is 5% at maximum. An absorbent core where said dimensions differ up to 5% is still considered as geometrically symmetrical. Said dimensions of the pad may also differ up to 5% and still be considered as geometrically symmetrical.

In a specific embodiment, the attaching arrangements are provided on the wearer-facing side of the pad and are intended to be attached to the side of the belt oriented away from the wearer.

In particular, the attaching arrangements comprise hook elements provided on each corner of the longitudinal ends of the pad intended to be attached to loop elements provided on a belt. Alternatively, the attaching arrangements may comprise loop elements provided on each corner of longitudinal ends of the pad intended to be attached to hook elements provided on a belt. Optionally, the hook elements may be attached to the belt itself formed of non-woven (i.e. the belt itself then provides loop elements).

Alternatively, the attaching arrangements comprise adhesive elements provided on each corner of the longitudinal ends of the pad intended to be attached to landing zones provided on a belt.

In an alternative embodiment, the attaching arrangements are provided on tabs extending outwardly from each of the waist portions of the pad.

In order to distinguish the high absorption capacity half from the lower absorption capacity half of the core, the longitudinal half of the pad with higher absorption capacity may be provided with indicating means to enable the wearer to use the pad in a desired manner (i.e. to place the pad so that the half having the higher absorption capacity is either at the rear end or the front end of the article when used).

Alternatively, the longitudinal half of the pad with lower absorption capacity may be provided with indicating means.

The indicating means may comprise visually indicating means or tactile indicating means suitable for all kinds of persons, including blind persons.

The present invention also provides an absorbent article comprising said absorbent pad described above and a separate belt to which the pad is releasably attached.

Since the pad and core in the article according to the invention as described above are both symmetrical in longitudinal direction and transversal direction and especially since the thickness of the core is same on the same distance from the transversal centre line (measured perpendicular to the transversal centre line), the pad can be attached to the wearer via a separate belt with any of its two halves in forward/reward direction depending on the specific needs of the user. With either end placed forward or reward, the article will be comfortable for the wearer during use due to the unnoticeable difference in profile of the two longitudinal halves.

### BRIEF DESCRIPTION OF THE DRAWINGS

The invention will now be explained in more detail with reference to certain nonlimiting embodiments and with reference to the accompanying drawings, in which:
Fig. 1 is a plan view showing an absorbent article with an absorbent pad attached to a separate belt, according to one embodiment of the present invention.
Fig. 2A is a plan view showing the absorbent pad of the present invention shown in Fig. 1.
Fig. 2B is a plan view showing an absorbent pad according to another embodiment of the present invention intended to be attached to a separate belt.
Fig. 3 is a perspective view showing a separate belt intended to be attached to the absorbent pad of the present invention.

### DETAILED DESCRIPTION OF THE INVENTION

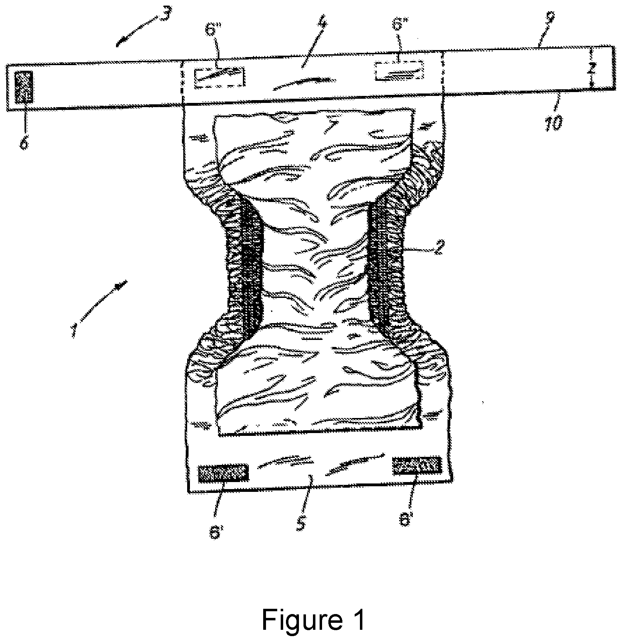

Embodiments of the present invention will be described below with reference to the drawings. Fig. 1 shows an absorbent article 1 which consists of an absorbent pad 2 and a separate belt 3. The belt 3 is a continuous belt 3 releasably attached to the two longitudinal ends 4, 5 of the absorbent pad 2 at substantially central portion(s) of the belt 3 when the article is put on a wearer.

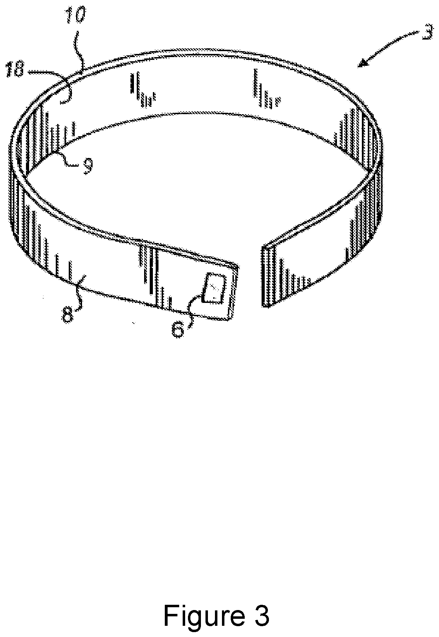

As shown in Fig. 3, at one end of the belt 3 there is provided a flexible strip 6 of hook elements, of the hook and loop type of fastening means, which can either be secured to the outer surface 8 of the belt 3 or to a loop material arranged on the surface 8. The belt 3 is elongated having a rectangular shape comprising two laterally spaced edges 9 and 10 between which the strip 6 will be attached, but other shapes are also conceivable.

In particular, a non-woven material may be used for either one or both sides of the belt 3, said non-woven material being of a type to which the hook elements of the strip 6 can be releasably attached.

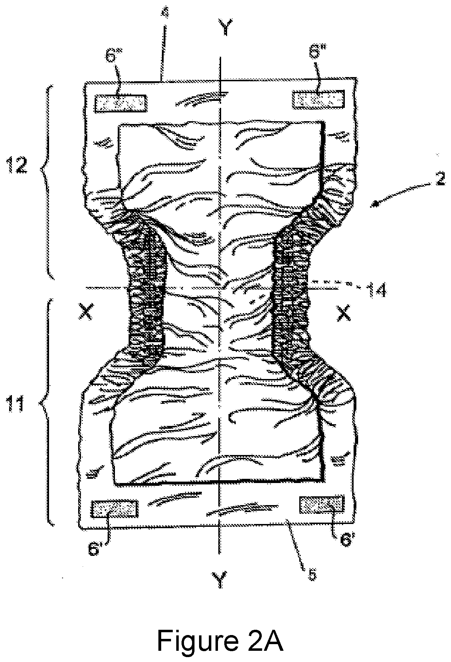

Fig. 2A shows the absorbent pad 2 of the absorbent article 1. On each corner of the pad 2, there is a strip 6' or 6". Fig. 2A shows four strips, with two strips 6' arranged at the end 4 and the other two strips 6" arranged at the other end 5. These strips 6', 6" are used to be releasably attached to either surface 8 or 18 of the belt 3. In particular, these strips 6', 6" are formed of the same materials as that of the strip 6 on the belt 3, such as hook elements.

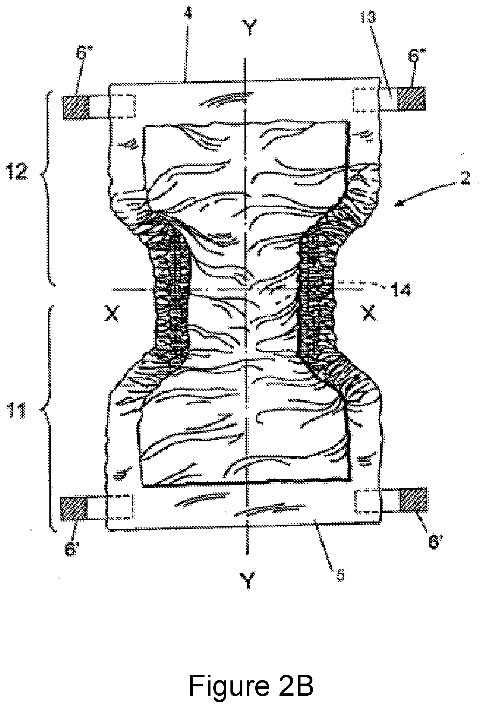

Fig. 2B shows another alternative embodiment of the absorbent pad 2. Each of the waist portions of the pad 2 is provided with two tabs 13, with one tab extending outward laterally from each edge of the waist portion. On the wearer-facing side of each of the tabs, a strip 6' or 6" is provided. These strips 6', 6" are formed of the same material as that of the strip 6 on the belt 3, such as hook elements. These strip 6', 6" are used to be releasably attached to the outer surface 8 of the belt. Alternatively the strips 6' or 6"are provided on the opposite side of the tabs 13. In this case the strips 6'; 6" are used to be releasably attached to the wearer-facing side 18 of the belt.

As it best can be seen from Fig. 2A and 2B, the pad 2 is of a geometrically symmetrical structure which is symmetrical both along the longitudinal centre line Y-Y and the transverse centre line X-X so as to divide the pad into two longitudinal halves, also referred to as first longitudinal half and second longitudinal half.

The absorbent pad shown in Fig. 2A and 2B comprises a chassis having a liquid-permeable topsheet, a liquid-impermeable backsheet and an absorbent core 14 located there between.

The topsheet of the absorbent pad is the layer which lies in contact with the wearer's body when the pad is in use. As such, it should be soft, non-irritating and comfortable against the skin, and bodily fluid should be able to pass through it without hindrance. The topsheet can consist of a nonwoven material, e.g. spunbond, meltblown, carded, hydroentangled, wetlaid etc. Suitable nonwoven materials can be composed of natural fibers, such as wood pulp or cotton fibres, man-made fibres, such as polyester, polyethylene, polypropylene, viscose etc, or from a mixture of natural and man-made fibres. The topsheet may further be composed of tow fibres, which may be bonded to each other in a bonding pattern. Further examples of materials suitable for topsheets are porous foams, apertured plastic films etc.

The backsheet of the absorbent pad is the layer which lies furthest from the wearer's body when the pad is used. To protect the wearer's garments from soiling, it should be liquid-impermeable, but is desirably gas-permeable (i.e. breathable) to allow air and vapour to pass in and out of the article so that the warm, damp conditions which can arise in the pad are reduced. Typically, the backsheet is of a liquid-impervious material, such as a thin plastic film, e g a polyethylene or polypropylene film, a nonwoven material coated with a liquid-impervious material, a hydrophobic nonwoven material, which resists liquid penetration or a laminate comprising plastic film(s) and nonwoven material(s). Examples of breathable backsheet materials are porous polymeric films, nonwoven laminates from spunbond and meltblown layers, laminates from porous polymeric film(s) and nonwoven(s).

The absorbent core 14 of the absorbent pad acts to receive and contain liquid and other bodily exudates. As such, it typically comprises absorbent material. Examples of commonly occurring absorbent materials are cellulosic fluff pulp, tissue layers, highly-absorbent polymeric materials (so called superabsorbents, SAP), absorbent foam materials, absorbent nonwoven materials or the like. It is common to combine cellulosic fluff pulp with superabsorbents in an absorbent body.

The absorbent core 14 may comprise one or more layers which are designed to improve the handling of bodily waste.

The topsheet and backsheet generally have similar extensions in the plane of the article, while the absorbent core 14 generally has an extension which is somewhat smaller. The topsheet and backsheet are joined to one another around the periphery of the absorbent core, so that the core 14 is enclosed within the envelope formed by the topsheet and the backsheet. The absorbent core is centrally located in the absorbent pad. The topsheet and backsheet may be joined to one another by any means common in the art, e.g. ultrasonic welding, thermal welding or gluing.

The term "absorbent pad" is to be understood as meaning an article selected from the group consisting of diapers, male or female incontinence guards, pant diapers, etc. Such pads are used for the absorption of bodily exudates, such as blood, urine, sweat and faeces.

In the embodiments of the present invention, as shown in Fig. 2A and Fig. 2B, the absorbent core 14 is also symmetrical both along the longitudinal centre line Y-Y and along the transverse centre line X-X of the absorbent pad so that the absorbent core 14 is also divided into two longitudinal halves, the first longitudinal half 11 and the second longitudinal half 12. It is important that the thickness of the core 14 is substantially the same on the same distance from the transversal centre line (measured perpendicular to the transversal centre line) before it is wetted by the bodily exudates. After the article is wetted, the two longitudinal halves may have different thickness due to absorption of fluid.

In particular, the two halves of the absorbent core may contain different amount of superabsorbing material (SAP) thereby resulting in that the two halves of the core exhibit different absorption capacity. Specifically, the first longitudinal half 11 contains a larger amount of SAP than the second longitudinal half 12 so that the first half exhibits higher absorption capacity than the second half of the core.

Some examples of superabsorbing polymeric materials (SAP) are FAVOR SXM 9455 (from Evonic Stockhausen GmbH, Krefeld, Germany), FAVOR SXM 9410 (from Evonic Stockhausen GmbH, Krefeld, Germany), Sanwet IM 930 (from San-Dia Polymers, Tokyo, Japan), HySorb B 7160 S (from BASF, Ludwigshafen, Germany) and HySorb R 7061 (from BASF, Ludwigshafen, Germany).

In specific embodiments, the amount of SAP contained in the first half and second half of the absorbent core 14, respectively, may be set in such a manner that the absorption capacity ratio of the first half to the second half is 80:20, 60:40 or 55:45.

The absorption capacity of the absorbent pad (including both the first longitudinal half and the second longitudinal half of the pad) is generally within the range of from 500 to 4 000 ml (measured according to Rothwell ISO 11941-1).

More specifically, the absorption capacity of the first longitudinal half is generally within the range of from 275 to 3 200 ml (measured according to Rothwell ISO 11941-1) and the absorption capacity of the second longitudinal half is generally within the range of from 100 to 1 800 ml (measured according to Rothwell ISO 11941-1), provided that the first longitudinal half has a higher absorption capacity than the second longitudinal half, e.g. the absorption capacity ratio of the first longitudinal half to the second longitudinal half of the absorbent core may be 80:20, in particular 60:40 or more particularly 55:45. It is within the knowledge of a person skilled in the art to select a suitable superabsorbent polymeric material and the amount thereof needed in order to obtain the desired absorption capacity.

Herein, with regard to the core 14, the term "geometrically symmetrical" means that not only the absorption core has a symmetrical profile in the X-Y plane, but also the thickness of the core is substantially the same on the same distance from the transversal centre line X-X, measured perpendicular to the line X-X, before the core is wetted. After the core has been wet the half of the core with higher absorption capacity may be thicker than the other half of the core due to absorption of more fluid.

An absorption core that is "geometrically symmetrical" in the X-Y plane can vary slightly due to tolerances in production process, especially when compressing the absorption core (rolling out effects). According to the present invention, a maximum difference of 5% of dimensions measured on equal perpendicular distance from the transversal centre line X-X (measuring points on the two halves mirrored in the X-X line) in the X-Y plane is within the scope of the invention and the core is still considered as geometrically symmetrical.

As used herein, "substantially the same thickness" refers to a maximum difference of 5% in thickness.

Since the absorbent pad is of a substantially symmetrical structure, it can be put on the wearer with any one end forward/reward to be attached to the belt. No limitation is applied to the orientation when the article is in use, which provides convenience and comfort for the wearer due to the symmetrical arrangement.

With the two halves of the article having different absorption capacity, the article can be used in different occasions by placing its high absorption capacity half in forward or reward direction for the specific need of the wearer, such as male/female, sleeping on his/her face or back, etc.

Due to the symmetrical structure of the absorbent pad 2 and the absorbent core 14, it is usually difficult for the user to distinguish the front portion from the rear potion, named the higher absorption capacity half and the lower absorption capacity half, by a single glimpse. To this end, the half of the article with higher absorption capacity may be provided with indicating means showing the user which half is the higher absorption capacity half. Alternatively, indicating means may be provided on the lower absorption capacity half.

The indicating means may comprise visually indicating means or tactile indicating means or both of them. The visually indicating means may be text, picture, trade mark, etc, which can also give the article a pleasurable appearance. The tactile indicating means can be projected or concaved objects provided on the outer surface of the absorbent pad. It is very helpful for blind persons to be able to easily distinguish the higher absorption capacity half from the lower absorption capacity half.

Although the invention is illustrated with attachment between the two ends of the belt as well as the attachment between the belt and the absorbent pad via hook & loop connections, other means of attaching such as by adhesive strips and landing zones, snap fitting, etc may also be used.

Although the embodiments of the present invention have been described above, the present invention is not limited to the above embodiments, but various changes and modification may be made therein within the scope of the technical concept described in the scope of the claims.

## Claims

1. An absorbent pad having a chassis comprising a liquid-permeable topsheet and a liquid-impermeable backsheet, wherein the absorbent pad is geometrically symmetrical along its longitudinal and transversal centre lines and wherein the absorbent pad is provided with attaching arrangements by which the pad can be releasably attached to a separate belt adapted to be placed around the waist of the user,
**characterized in that** the absorbent pad further comprises an absorbent core interposed between the topsheet and the backsheet, which absorbent core is also geometrically symmetrical along the longitudinal and transversal centre lines of the pad and the absorbent core is divided into a first longitudinal half and a second longitudinal half along its longitudinal length,
wherein the absorbent core has a thickness which is substantially the same on the same distance from the transversal centre line measured perpendicular to the transversal centre line, before the core is wetted,
wherein the first longitudinal half has a higher absorption capacity than the second longitudinal half,
wherein the absorbent core comprises a superabsorbing polymeric material, and
wherein the first longitudinal half contains a different amount of superabsorbing polymeric material than the second longitudinal half, which causes the two longitudinal halves to exhibit different absorption capacity.

2. The absorbent pad according to claim 1, wherein the absorption capacity ratio of the first longitudinal half to the second longitudinal half of the core is 80:20.

3. The absorbent pad according to claim 1, wherein the absorption capacity ratio of the first longitudinal half to the second longitudinal half of the core is 60:40.

4. The absorbent pad according to claim 1, wherein the absorption capacity ratio of the first longitudinal half to the second longitudinal half of the core is 55:45.

5. The absorbent pad according to any one of claims 1-4, wherein the difference of the dimensions of the absorbent core measured on equal perpendicular distances from the transversal centre line is 5% at maximum.

6. The absorbent pad according to any one of claims 1-5, wherein attaching arrangements are provided on the side of the pad intended to face the wearer when used and intended to be releasably attached to a belt on the side of the belt oriented away from the wearer.

7. The absorbent pad according to any one of claims 1-6, wherein the attaching arrangements comprises (i) hook elements provided on each corner of the longitudinal ends of the pad intended to be attached to loop elements provided on a belt, or (ii) loop elements provided on each corner of the longitudinal ends of the pad intended to be attached to hook elements provided on a belt.

8. The absorbent pad according to any one of claims 1-6, wherein the attaching arrangements comprise adhesive elements provided on each corner of the longitudinal ends of the pad intended to be attached to landing zones provided on a belt.

9. The absorbent pad according to any one of claims 1-6, wherein the attaching arrangements are provided on tabs extending outwardly from each of the waist portions of the pad.

10. The absorbent pad according to any one of claims 1-9, wherein the longitudinal half of the pad with higher absorption capacity is provided with indicating means.

11. The absorbent pad according to any one of claims 1-9, wherein the longitudinal half of the pad with lower absorption capacity is provided with indicating means.

12. The absorbent pad according to claim 10 or 11, wherein the indicating means comprises visually indicating means or tactile indicating means.

13. An absorbent article comprising an absorbent pad and a separate belt to which the pad is releasably attached, **characterized in that** the absorbent pad is an absorbent pad according to any one of preceding claims.

## Patentansprüche

1. Saugfähiges Pad, das einen Chassis mit einer flüssigkeitsdurchlässigen Oberlage und einer flüssigkeitsundurchlässigen Rücklage aufweist, wobei das saugfähige Pad entlang seiner Längs- und Quermittellinie geometrisch symmetrisch ist und das saugfähige Pad mit Abringanordnungen versehen ist, durch die das Pad abnehmbar an einem getrennten Gurt angebracht werden kann, der um die Taille des Benutzers zu platzieren ist,
**dadurch gekennzeichnet, dass** das saugfähige Pad ferner einen saugfähigen Kern aufweist, der zwischen der Oberlage und der Rücklage eingefügt ist, wobei der saugfähige Kern ebenfalls entlang der Längs- und Quermittelinien des Pads geometrisch symmetrisch ist und der saugfähige Kern entlang seiner Länge in Längsrichtung in eine erste Hälfte in Längsrichtung und eine zweite Hälfte in Längsrichtung unterteilt ist,
wobei der saugfähige Kern eine senkrecht zu der Quermittellinie gemessene Dicke aufweist, die vor Benetzen des Kerns im Wesentlichen bei dem gleichen Abstand von der Quermittellinie die gleiche ist,
die erste Hälfte in Längsrichtung eine höhere Aufnahmekapazität als die zweite Hälfte in Längsrichtung aufweist,
der saugfähige Kern ein superabsorbierendes Polymermaterial aufweist und
die erste Hälfte in Längsrichtung eine andere Menge an superabsorbierendem Polymermaterial aufweist als die zweite Hälfte in Längsrichtung, was verursacht, dass die zwei Hälften in Längsrichtung unterschiedlichen Aufnahmekapazitäten zeigen.

2. Saugfähiges Pad nach Anspruch 1, bei dem das Aufnahmekapazitätsverhältnis des Kerns von der ersten Hälfte in Längsrichtung zu der zweiten Hälfte in Längsrichtung 80:20 ist.

3. Saugfähiges Pad nach Anspruch 1, bei dem das Aufnahmekapazitätsverhältnis des Kerns von der ersten Hälfte in Längsrichtung zu der zweiten Hälfte in Längsrichtung 60:40 ist.

4. Saugfähiges Pad nach Anspruch 1, bei dem das Aufnahmekapazitätsverhältnis des Kerns von der ersten Hälfte in Längsrichtung zu der zweiten Richtung in Längsrichtung 55:45 ist.

5. Saugfähiges Pad nach einem der Ansprüche 1-4, bei dem der bei gleichen senkrechten Abständen von der Quermittellinie gemessene Unterschied der Abmessungen des saugfähigen Kerns maximal 5 % beträgt.

6. Saugfähiges Pad nach einem der Ansprüche 1-5, bei dem Anbringanordnungen auf der Seite des Pads bereitgestellt sind, die dazu vorgesehen sind, bei Verwendung dem Träger zugewandt zu sein, und dazu vorgesehen sind, abnehmbar an einem Gurt auf der Seite des Gurts angebracht zu sein, die von dem Träger weg gerichtet ist.

7. Saugfähiges Pad nach einem der Ansprüche 1-6, bei dem die Anbringanordnungen (i) Hakenelemente, die an jeder Ecke der Enden in Längsrichtung des Pads bereitgestellt sind, die dazu vorgesehen sind, an Schlaufenelementen angebracht zu sein, die an einem Gurt bereitgestellt sind, oder (ii) Schlaufenelemente aufweisen, die an jeder Ecke der Enden in Längsrichtung des Pads bereitgestellt sind, die dazu vorgesehen sind, an Hakenelementen angebracht zu sein, die an einem Gurt bereitgestellt sind.

8. Saugfähiges Pad nach einem der Ansprüche 1-6, bei dem die Anbringanordnungen Haftelemente aufweisen, die an jeder Ecke der Enden in Längsrichtung des Pads bereitgestellt sind und dazu vorgesehen sind, an Landezonen angebracht zu sein, die an einem Gurt bereitgestellt sind.

9. Saugfähiges Pad nach einem der Ansprüche 1-6, bei dem die Anbringanordnungen an Laschen vorgesehen sind, die sich von jeder der Taillenabschnitte des Pads nach außen erstrecken.

10. Saugfähiges Pad nach einem der Ansprüche 1-9, bei dem die Hälfte in Längsrichtung des Pads mit einer höheren Aufnahmekapazität mit einem Anzeigemittel versehen ist.

11. Saugfähiges Pad nach einem der Ansprüche 1-9, bei dem die Hälfte in Längsrichtung des Pads mit einer niedrigeren Aufnahmekapazität mit einem Anzeigemittel versehen ist.

12. Saugfähiges Pad nach Anspruch 10 oder 11, bei dem das Anzeigemittel ein visuelles Anzeigemittel oder fühlbares Anzeigemittel aufweist.

13. Saugfähiger Artikel mit einem saugfähigen Pad und einem getrennten Gurt, an dem das Pad abnehmbar angebracht ist, **dadurch gekennzeichnet, dass** das saugfähige Pad ein saugfähiges Pad nach einem der vorstehenden Ansprüche ist.

## Revendications

1. Serviette absorbante ayant un châssis comprenant une feuille supérieure perméable aux liquides et une feuille arrière imperméable aux liquides, dans laquelle la serviette absorbante est géométriquement symétrique le long de ses lignes médianes longitudinale et transversale et dans laquelle la serviette absorbante est pourvue d'agencements de fixation par lesquels la serviette peut être attachée de façon amovible à une ceinture distincte adaptée pour être placée autour de la taille de l'utilisateur,
**caractérisée en ce que** la serviette absorbante comprend en outre une âme absorbante interposée entre la feuille supérieure et la feuille arrière, laquelle âme absorbante est également géométriquement symétrique le long des lignes médianes longitudinale et transversale de la serviette et l'âme absorbante est divisée en une première moitié longitudinale et une seconde moitié longitudinale le long de sa longueur longitudinale, et
dans laquelle l'âme absorbante a une épaisseur qui est substantiellement identique sur la même distance de la ligne médiane transversale mesurée perpendiculairement à la ligne médiane transversale, avant que l'âme ne soit mouillée,
dans laquelle la première moitié longitudinale a une capacité d'absorption supérieure à la seconde moitié longitudinale,
dans laquelle l'âme absorbante comprend un matériau polymère superabsorbant, et
dans laquelle la première moitié longitudinale contient une quantité différente de matériau polymère superabsorbant que la seconde moitié longitudinale, ce qui amène les deux moitiés longitudinales à présenter une capacité d'absorption différente.

2. Serviette absorbante selon la revendication 1, dans laquelle le rapport de capacité d'absorption de la première moitié longitudinale à la seconde moitié longitudinale de l'âme est de 80:20.

3. Serviette absorbante selon la revendication 1, dans laquelle le rapport de capacité d'absorption de la première moitié longitudinale à la seconde moitié longitudinale de l'âme est de 60:40.

4. Serviette absorbante selon la revendication 1, dans laquelle le rapport de capacité d'absorption de la première moitié longitudinale à la seconde moitié longitudinale de l'âme est de 55:45.

5. Serviette absorbante selon l'une quelconque des revendications 1-4, dans laquelle la différence des dimensions de l'âme absorbante mesurée sur des distances perpendiculaires égales à partir de la ligne médiane transversale est de 5% au maximum.

6. Serviette absorbante selon l'une quelconque des revendications 1-5, dans laquelle des agencements de fixation sont prévus sur le côté de la serviette destinés à faire face au porteur lorsqu'elle est utilisée et destinés à être fixés de manière amovible à une ceinture du côté de la ceinture orienté à l'opposé du porteur.

7. Serviette absorbante selon l'une quelconque des revendications 1-6, dans laquelle les agencements de fixation comprennent (i) des éléments de crochet prévus sur chaque coin des extrémités longitudinales de la serviette destinés à être attachés à des éléments en boucle prévus sur une ceinture, ou (ii) des éléments en boucle prévus sur chaque coin des extrémités longitudinales de la serviette destinés à être fixés à des éléments de crochet prévus sur une ceinture.

8. Serviette absorbante selon l'une quelconque des revendications 1-6, dans laquelle les agencements de fixation comprennent des éléments adhésifs prévus sur chaque coin des extrémités longitudinales de la serviette destinés à être attachés à des zones de réception prévues sur une ceinture.

9. Serviette absorbante selon l'une quelconque des revendications 1-6, dans laquelle les agencements de fixation sont prévus sur des languettes s'étendant vers l'extérieur à partir de chacune des parties de la ceinture de la serviette.

10. Serviette absorbante selon l'une quelconque des revendications 1-9, dans laquelle la moitié longitudinale de la serviette avec une capacité d'absorption supérieure est pourvue de moyens d'indication.

11. Serviette absorbante selon l'une quelconque des revendications 1-9, dans laquelle la moitié longitudinale de la serviette avec une capacité d'absorption inférieure est pourvue de moyens d'indication.

12. Serviette absorbante selon la revendication 10 ou 11, dans laquelle les moyens d'indication comprennent des moyens d'indication visuelle ou des moyens d'indication tactile.

13. Article absorbant comprenant une serviette absorbante et une ceinture séparée à laquelle la serviette est fixée de manière amovible, **caractérisé en ce que** la serviette absorbante est une serviette absorbante selon l'une quelconque des revendications précédentes.
